(19) Europäisches Patentamt / European Patent Office / Office européen des brevets

(11) **EP 4 141 100 A1**

(12) **EUROPEAN PATENT APPLICATION**
published in accordance with Art. 153(4) EPC

(43) Date of publication:
**01.03.2023 Bulletin 2023/09**

(51) International Patent Classification (IPC):
*C12M 3/00* (2006.01)   *C12M 1/34* (2006.01)
*C12M 1/12* (2006.01)   *C12M 1/04* (2006.01)

(21) Application number: **21811985.7**

(22) Date of filing: **26.01.2021**

(86) International application number:
**PCT/CN2021/073796**

(87) International publication number:
**WO 2021/238286 (02.12.2021 Gazette 2021/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.05.2020 CN 202010457769**

(71) Applicant: **Alit Biotech (Shanghai) Co., Ltd.
Shanghai 201615 (CN)**

(72) Inventors:
• **LIU, Yu
  Shanghai 201613 (CN)**
• **CHEN, Rui
  Shanghai 201613 (CN)**

(74) Representative: **Wang, Bo
Panovision IP
Ebersberger Straße 3
85570 Markt Schwaben (DE)**

(54) **GAS INTRODUCING DEVICE FOR BIOREACTOR**

(57)   The present disclosure discloses a ventilation device for a bioreactor, which has a tank containing a mixture of animal cells and liquids. The ventilation device comprises: a gas distributor, which is configured to be immersed in the mixture of the tank, and includes a distributor body and a diaphragm supported by the distributor body, and the distributor body is provided with a ventilation slot, the diaphragm is configured to dissolve a gas in the ventilation tank into the mixture without bubbles; a gas inlet unit, which is in fluid communication with the ventilation slot and transmits an inlet gas to the ventilation slot, and a gas outlet unit, which is in fluid communication with the ventilation slot and discharges an undissolved gas in the ventilation slot from the distributor body. The ventilation device greatly improves a monitoring level of the bioreactor on an oxygen consumption rate in a biological process.

FIG. 1

**Description**

**TECHNICAL FIELD**

The present disclosure generally relates to the field of bioreactors. More specifically, the present disclosure relates to a ventilation device for a bioreactor.

**BACKGROUND**

In the design of bioreactors for animal cells, the solubility of oxygen in the culture medium has always been one of the most important design parameters. In general, the rate of oxygen transmission is greater than that of animal cells to maintain the normal metabolic level of animal cells. Deep ventilation bioreactor usually makes the air bubbles as small as possible by adopting various methods, in order to improve the specific surface area of mass transfer under the unit amount of gas and increase the efficiency of oxygen transfer. However, the study found that the smaller the bubbles, the greater the shear force generated by the bubble rupture in the rising process, which may have an irreversible impact on the vulnerable animal cells.

At present, most disposable bioreactors use the combination of centimeter-scale bubble distributors and micron-scale bubble distributors. The bubble diameter generated by the centimeter-scale bubble distributor is larger and the specific surface area of mass transfer is smaller. Therefore, the centimeter-scale bubble distributor has a better ability to discharge carbon dioxide, but a poor ability to transfer oxygen, and it is generally used to disperse excess carbon dioxide bubbles dissolved in the culture medium. The micron-scale bubble distributor is usually made by a sintering method of metal or plastic materials, and its minimum pore diameter may be controlled to 2-20um. Under the same gas flow conditions, the micron-scale bubble distributor produces a large number of bubbles and effectively improves the specific surface area of mass transfer. However, too small bubbles produced by the micron-scale bubble distributor causes significant damage to animal cells (especially stem cells). Therefore, in the design of the bioreactor for animal cells, the contradiction between oxygen transfer and bubble shear force is more prominent.

Therefore, the bubble-free design of a mass transfer system is the direction of development in the future. However, the existing bubble-free bioreactor adopts a mode of mass transfer in the external circulation of a reactor, uses a peristaltic pump to pump the culture medium out of the culture system, and increases the shear damage of the peristaltic pump to animal cells. In addition, the existing bioreactors are mainly stainless-steel tanks, which need damp-heat sterilization in the process of use, so it is difficult to apply many newly developed microporous membrane materials.

On the other hand, in the process control of bioreactors, it is very important to monitor the oxygen consumption rate of animal cells. The common practice is to pass the tail gas of the bioreactor into the gas mass spectrometer detector. The oxygen uptake rate (OUR) consumed by animal cells is inferred by comparing the inlet gas flow and its gas composition, and the outlet gas flow and its gas composition. However, in the process of gas outlet, the non-liquid area above the bioreactor may form a tail gas buffer zone, making the calculation of OUR invalid and it is difficult to obtain the real-time value of OUR. In addition, the gas mass spectrometer detector is very expensive, which greatly increases the production cost.

**SUMMARY**

One of the purposes of the present disclosure is to provide a ventilation device capable of overcoming at least one defect in the prior art.

The first aspect of the present disclosure relates to a ventilation device for a bioreactor, wherein the bioreactor has a tank containing a mixture of animal cells and liquids, and the ventilation device includes:

a gas distributor, which is configured to be immersed in the mixture of the tank and includes a distributor body and a diaphragm supported by the distributor body, wherein the distributor body is provided with a ventilation slot, and the diaphragm is configured to dissolve a gas in the ventilation slot into the mixture without bubbles;

a gas inlet unit, which is in fluid communication with the ventilation slot and transmits an inlet gas to the ventilation slot, and

a gas outlet unit, which is in fluid communication with the ventilation slot and discharges an undissolved gas in the ventilation slot from the distributor body.

In some embodiments, the ventilation slot is arranged on a wall of the distributor body and is configured to guide the inlet gas to flow on an entire outer surface of the wall.

In some embodiments, the wall includes a top flange, a bottom flange, and one or more protrusions between the top flange and the bottom flange, and the top flange, the bottom flange, and the protrusions all protrude radially outward from an outer surface of the wall.

In some embodiments, the top flange, the bottom flange, and/or the protrusions form a support frame supporting the diaphragm.

In some embodiments, the ventilation slot is formed in a space between the outer surface of the wall and a side surface of adj acent protrusions, and/or in a space between the outer surface of the wall, a side surface of the protrusions and a side surface of the top flange, and/or in a space between the outer surface of the wall, the side surface of the protrusions and a side surface of the bottom flange.

In some embodiments, the ventilation slot is in fluid communication with the gas inlet unit through a gas inlet of the distributor body and is in fluid communication with the gas outlet unit through a gas outlet of the distributor body.

In some embodiments, the gas inlet and the gas outlet are tubular.

In some embodiments, the ventilation grooves are mainly winding in the horizontal direction on the entire surface of the wall.

In some embodiments, the ventilation slot presents a serpentine shape winding mainly along a horizontal direction on the entire outer surface of the wall.

In some embodiments, the ventilation slot presents a serpentine shape winding mainly along a vertical direction on the entire outer surface of the wall.

In some embodiments, the diaphragm is arranged on the outer surface of the wall and covers the ventilation slot.

In some embodiments, the diaphragm is fixed to radial outer surfaces of the top flange, the bottom flange, and/or the protrusions of the wall.

In some embodiments, the diaphragm is in a form of a dense membrane or a microporous membrane.

In some embodiments, a pore size of the microporous membrane is configured to ventilate the membrane without bubbles under a certain pressure.

In some embodiments, the pore size of the microporous membrane is less than 0.05 $\mu$m.

In some embodiments, the pore size of the microporous membrane is one or more of 0.01 $\mu$m, 0.05 um, 0.10 $\mu$m, 0.20 $\mu$m, and 0.04 $\mu$m.

In some embodiments, a thickness of the dense membrane is between 50 $\mu$m to 500 $\mu$m.

In some embodiments, the diaphragm is made of silica gel, polydimethylsiloxane (PDMS), polycarbonate track-etch (PCTE), polyethylene terephthalate (PETE), polytetrafluoroethylene (PTFE), polypropylene (PP), polycarbonate (PC), nylon, polyethersulfone (PES), or sintered porous material.

In some embodiments, the diaphragm is processed with hydrophilic and positive charge or negative charge treatment so that it is not easy to be blocked due to being adsorbed by animal cells.

In some embodiments, the gas inlet unit is in fluid communication with the ventilation slot through a gas inlet pipeline, the gas inlet unit includes a gas flow component regulator arranged on the gas inlet pipeline and electrically connected to a controller, the gas flow component regulator is configured to maintain a total flow of the inlet gas at a constant value and adjust a component proportion of the inlet gas according to a command of the controller.

In some embodiments, the gas flow component regulator is any one of a mass flowmeter or a gas-proportion regulating valve.

In some embodiments, the inlet gas includes air, oxygen, and carbon dioxide, or includes nitrogen, oxygen, and carbon dioxide.

In some embodiments, the gas outlet unit is in fluid communication with the ventilation slot through the gas outlet pipeline, the gas outlet unit includes a first dissolved oxygen electrode arranged in the gas outlet pipeline and electrically connected to the controller, and the first dissolved oxygen electrode is configured to detect a dissolved oxygen concentration value in the gas outlet pipeline.

In some embodiments, the gas outlet unit further includes a pressure control valve and a sensor arranged in the gas outlet pipeline and electrically connected to the controller to control gas pressure in the ventilation slot.

In some embodiments, the gas pressure in the ventilation slot is maintained between 0.01Mpa-0.1Mpa.

In some embodiments, the ventilation device further includes a solution monitoring unit, which includes a second dissolved oxygen electrode arranged in the mixture and electrically connected to the controller, the second dissolved oxygen electrode is configured to detect the dissolved oxygen concentration value in the mixture.

In some embodiments, the solution monitoring unit includes a pH electrode arranged in the mixture and electrically connected to the controller, and the pH electrode is configured to detect a pH value in the mixture.

In some embodiments, the controller is configured to monitor and adjust an oxygen uptake rate OUR consumed by the animal cells in the tank in real-time through the dissolved oxygen concentration value Cout detected by the first dissolved oxygen electrode and the dissolved oxygen concentration value Csensor detected by the second dissolved oxygen electrode.

In some embodiments, the oxygen uptake rate OUR consumed by the animal cells in the tank is determined by a calculation formula of

$$OUR = (Cout-Csensor)*KLa,$$

where KLA is a mass transfer coefficient of a gas distributor.

In some embodiments, the mass transfer coefficient of the gas distributor is related to a rotational speed of a motor in the tank which drives the circulating flow of the mixture.

In some embodiments, the controller is configured to adjust a proportion of carbon dioxide in the inlet gas of the gas flow component regulator through a pH value detected by a pH electrode, so as to adjust the pH value of the mixture.

The second aspect of the present disclosure relates to a ventilation device for a bioreactor, wherein the bioreactor has a tank containing a mixture of animal cells and liquids, and the ventilation device includes:

    a gas distributor, which is configured to be immersed in the mixture of the tank and is configured to dissolve an inlet gas into the mixture without bubbles;

a gas inlet unit, which is in fluid communication with the gas distributor and transmits an inlet gas to the gas distributor, the gas inlet unit including a gas flow component regulator, wherein the gas flow component regulator is configured to maintain a total flow of the inlet gas at a constant value and adjust the component proportion of the inlet gas.as intake gas,

a gas outlet unit, which is in fluid communication with the gas distributor and discharges an undissolved gas in the gas distributor from the distributor body, wherein the gas outlet unit includes a first dissolved oxygen electrode configured to detect a dissolved oxygen concentration value in the undissolved gas;

a solution monitoring unit, which includes a second dissolved oxygen electrode configured to detect the dissolved oxygen concentration value in the mixture; and

a controller, which is configured to monitor and adjust an oxygen uptake rate OUR consumed by animal cells in the tank in real-time through the dissolved oxygen concentration value Cout detected by the first dissolved oxygen electrode and the dissolved oxygen concentration value Csensor detected by the second dissolved oxygen electrode.

In some embodiments, the gas flow component regulator is any one of a mass flowmeter or a gas-proportion regulating valve.

In some embodiments, the inlet gas includes air, oxygen, and carbon dioxide, or includes nitrogen, oxygen, and carbon dioxide.

In some embodiments, the gas outlet unit further comprises a pressure control valve configured to control gas pressure in the gas distributor and a sensor.

In some embodiments, the gas pressure in the gas distributor is maintained between 0.01Mpa-0.1Mpa.

In some embodiments, the solution monitoring unit comprises a pH electrode arranged in the mixture and electrically connected to the controller, and the pH electrode is configured to detect the pH value in the mixture.

In some embodiments, the oxygen uptake rate OUR consumed by the animal cells in the tank is determined by a calculation formula of

$$OUR = (Cout - Csensor) * KLa,$$

where KLA is a mass transfer coefficient of the gas distributor.

In some embodiments, the mass transfer coefficient of the gas distributor is related to a rotational speed of a motor in the tank which drives the circulating flow of the mixture.

In some embodiments, the gas controller is configured to adjust a proportion of carbon dioxide in the inlet gas of the gas flow component regulator through a pH value detected by a pH electrode, so as to adjust a pH value of the mixture.

In some embodiments, the gas distributor includes a distributor body and a diaphragm supported by the distributor body, the distributor body being provided with a ventilation slot, and the diaphragm is configured to dissolve a gas in the ventilation slot into the mixture without bubbles;

In some embodiments, the diaphragm is in a form of a dense membrane or a microporous membrane.

In some embodiments, a pore size of the microporous membrane is configured to ventilate the membrane without bubbles under a certain pressure.

In some embodiments, the pore size of the microporous membrane is less than 0.05 $\mu$m.

In some embodiments, the pore size of the microporous membrane is one or more of 0.01 $\mu$m, 0.05 um, 0.10 $\mu$m, 0.20 $\mu$m, and 0.04 $\mu$m.

In some embodiments, a thickness of the dense membrane is between 50 $\mu$m to 500 $\mu$m.

In some embodiments, the diaphragm is made of silica gel, polydimethylsiloxane (PDMS), polycarbonate track-etch (PCTE), polyethylene terephthalate (PETE), polytetrafluoroethylene (PTFE), polypropylene (PP), polycarbonate (PC), nylon, polyethersulfone (PES), sintered porous material, or the like.

In some embodiments, the diaphragm is processed with hydrophilic and positive charge or negative charge treatment so that it is not easy to be blocked due to being adsorbed by animal cells.

The other characteristics and advantages of this public theme technology will be explained in the description below, and partly will be obvious from the description, or can be learned by practicing the theme technology of this public. The advantages of the subject technology of the present disclosure may be realized and obtained through the structure especially pointed out in the written disclosure, we claim, and the drawings.

It should be understood that the aforementioned general description and the following detailed description are all exemplary and illustrative, and the purpose of providing further description of the claimed subject technology of the present disclosure.

**BRIEF DESCRIPTION OF THE DRAWINGS**

After reading the following specific embodiments in combination with the drawings, various aspects of the present disclosure will be better understood. In the drawings:

FIG. 1 is a schematic diagram illustrating an exemplary ventilation device according to some embodiments of the present disclosure;

FIG. 2 is an exploded perspective diagram of a gas distributor of the ventilation device shown in FIG. 1;

FIG. 3A is a schematic diagram illustrating an exemplary diaphragm of the ventilation device shown in FIG. 1; FIG. 3B is another schematic diagram illustrating an exemplary diaphragm of the ventilation device shown in FIG. 1; and

FIG. 4 is a schematic diagram illustrating an exemplary relationship between a mass transfer coefficient of the diaphragm, a motor speed and a total ventilation volume.

## DETAILED DESCRIPTION

The present disclosure will be described below with reference to the drawings, which show several embodiments of the present disclosure. However, it should be understood that the present disclosure may be presented in a variety of different ways and is not limited to the embodiments described below. In fact, the embodiments described below are intended to make the disclosure of the present disclosure more complete and fully explain the scope of protection of the present disclosure to those skilled in the art. It should also be understood that the embodiments disclosed in the present disclosure can be combined in various ways to provide more additional embodiments.

It should be understood that in all the drawings, the same reference numerals refer to the same elements. In the drawings, the dimensions of some features can be deformed for clarity.

It should be understood that the use of words in the specification are only used to describe specific embodiments and are not intended to limit the present disclosure. Unless otherwise defined, all terms (including technical terms and scientific terms) used in the present disclosure have meanings commonly understood by those skilled in the art. Well-known functions or constructions may not be described in detail for brevity and/or clarity.

As shown in the present disclosure and claim, unless the context clearly prompts the exception "a", "an", "this", and/or "the" is not specifically singular, and the plural may be included. The terms "comprises," "comprising," "includes," "including" and/or "contain" used in the present disclosure indicate the existence of the claimed features, but do not exclude the existence of one or more other features. The term "and/or" used in the present disclosure includes any and all combinations of one or more of the relevant listed items. The terms "between X and Y" and "between about X and Y" used in the present disclosure shall be interpreted to include X and Y The term "between about X and Y" used in the present disclosure means "between about X and about y", and the term "from about X to Y" used in the present disclosure means "from about X to about Y".

In the present disclosure, when an element is said to be "on", "attached" to another element, "connected" to another element, "coupled" to another element, or "in contact" with another element, the element can be directly on another element, attached to another element, connected to another element, or in contact with another element, or there can be an intermediate element. In contrast, when an element is said to be "directly" on another element, "directly attached" to another element, "directly connected" to another element, "directly coupled" to another element, or "directly in contact" with another element, there will be no intermediate element. In the present disclosure, one feature is arranged to be "adjacent" to another feature, which refers to one feature that has a part overlapping with the adjacent feature or a part above or below the adjacent feature.

In the present disclosure, the spatial relationship terms such as "up", "down", "left", "right", "front", "back", "above", "below" and so on can explain the relationship between one feature and another feature in the drawings. It should be understood that the spatial relationship term includes different orientations of the device in use or operation in addition to the orientations shown in the drawings. For example, when the device in the drawings is reversed, the feature originally described as "below" other features can be described as "above" other features. The device can also be oriented in other ways (rotated 90 degrees or in other directions), and the relative spatial relationship will be explained accordingly.

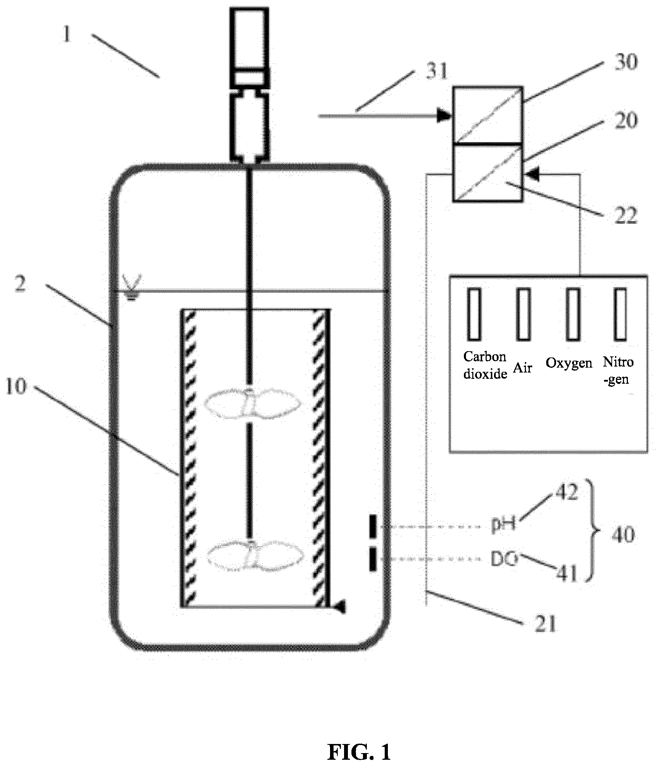

FIG. 1 is a schematic diagram illustrating an exemplary ventilation device according to some embodiments of the present disclosure, the ventilation device 1 is used in various small bioreactors, such as a disposable animal cell bioreactor. The bioreactor includes a tank 2 to contain a mixture of animal cells and culture medium, or a mixture of animal cells and other liquids. The ventilation device 1 is used to transmit one or more gases, such as a mixture of oxygen, carbon dioxide, and air, or a mixture of oxygen, carbon dioxide, and nitrogen, to the mixture of animal cells and culture medium (or other liquids) in the tank 2. As shown in the figure, the ventilation device 1 may include a gas distributor 10, as well as a gas inlet unit 20 and a gas outlet unit 30 which are in fluid communication with the gas distributor 10. The gas inlet unit 20 is used to transmit a gas to the gas distributor 10. The gas distributor 10 is used to dissolve the gas received from the gas inlet unit 20 into the mixture of animal cells and culture medium (or other liquids) of the tank 2. The gas outlet unit 30 is used to discharge a remaining gas in the gas distributor 10 that is not dissolved in the mixture from the gas distributor 10. The ventilation device 1 may also include a solution monitoring unit 40 to monitor various parameters of the mixture in the tank 2, such as a dissolved oxygen concentration or a pH value. The ventilation device 1 may also include a controller (not shown) electrically connected to the gas inlet unit 20, the gas outlet unit 30, and the solution monitoring unit 40.

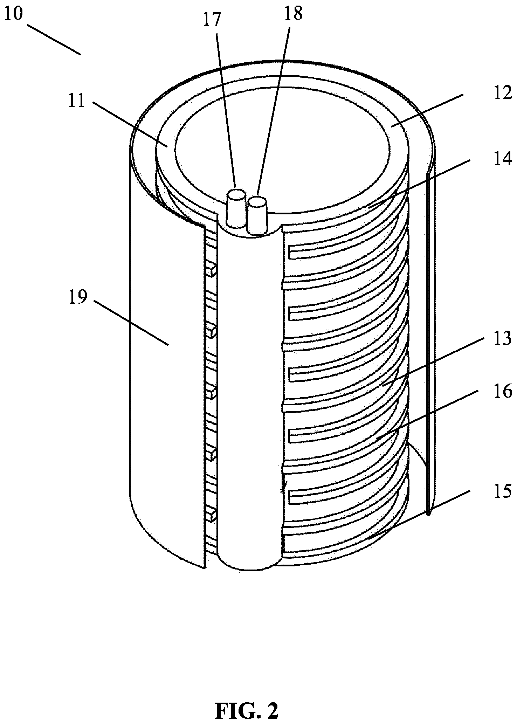

The gas distributor 10 is immersed in the mixture of animal cells and culture medium (or other liquids) of the tank 2. As shown in FIG. 2, the gas distributor 10 may be in a shape of a roughly hollow cylinder, and a cross-section of the cylinder may be circular, elliptical, triangular, quadrilateral, or any other shape. The gas distributor 10 may include a distributor body 11 and a diaphragm 19 supported by the distributor body 11. The distributor body 11 may transmit the gas received from the gas inlet unit 20 to one side of the entire diaphragm 19. The diaphragm 19 may transmit and dissolve the gas in a bubble-free manner into the mixture of animal cells and culture medium (or other liquids) located on another side.

As shown in the figure, the distributor body 11 may include a wall 12 and a ventilation slot 13 extending on the wall 12. The wall 12 may include a top flange 14, a bottom flange 15, and one or more protrusions 16 located between the top flange 14 and the bottom flange 15. The top flange 14, the bottom flange 15, and the protrusions 16 all protrude radially outward from an outer surface of the wall 12. The top flange 14, the bottom flange 15, and/or the protrusions 16 may form a support frame supporting the diaphragm 19, thereby keeping the diaphragm 19 flat.

The ventilation slot 13 is formed in a space between the outer surface of the wall 12 and a side surface of adj acent protrusions 16, or in a space between the outer surface of the wall 12, the side surface of the protrusions 16, and a side surface of the top flange 14, or in a space between the outer surface of the wall 12, the side surface of the protrusions 16 and a side surface of the bottom flange 15. The ventilation slot 13 is used to guide flow of the gas on the entire outer surface of the wall 12 to increase residence time of the gas in the gas distributor 10 and promote the gas mass transfer. The ventilation slot 13 is in fluid communication with the gas inlet unit 20 through the gas inlet 17 of the distributor body 11 and in fluid communication with the gas outlet unit 30 through the gas outlet 18 of the distributor body 11, thereby forming a gas flow passage through the gas inlet 17, the ventilation slot 13, and the gas outlet 18 on the distributor body 11. The gas may flow into the ventilation slot 13 from the gas inlet 17 and is dissolved into the mixture of animal cells and culture medium (or other liquids) of the tank 2 through the diaphragm 19 above the ventilation slot 13, and the undissolved gas flows out of the ventilation slot 13 through the gas outlet 18.

As shown in FIG. 2, the gas inlet 17 and the gas outlet 18 are tubular and may be arranged on the top flange 14. The ventilation slot 13 may present a serpentine shape winding mainly along a horizontal direction, winding from the gas inlet 17 of the top flange 14 on a part of the outer surface of the wall 12 gradually downward to the bottom flange 15, and winding from the bottom flange 15 gradually upward to the gas outlet 18 of the top flange 14 on another part of the outer surface of the wall 12. The gas inlet 17 and the gas outlet 18 may also be arranged at other positions of the wall 12, and the ventilation slot 13 may also be arranged on the wall 12 in various other patterns. For example, in one embodiment, the gas inlet 17 and the gas outlet 18 may be arranged on the top flange 14, the ventilation slot 13 presents a serpentine shape winding mainly along a vertical direction on the entire outer surface of the wall 12 and is in fluid communication with the gas inlet 17 and the gas outlet 18. In another embodiment, one of the gas inlet 17 and the gas outlet 18 is arranged on the top flange 14, and the other is arranged on the bottom flange 15, and the ventilation slot 13 may be present a spiral shape extending from bottom to top around the entire outer surface of the wall 12 and in fluid communication with the gas inlet 17 and the gas outlet 18.

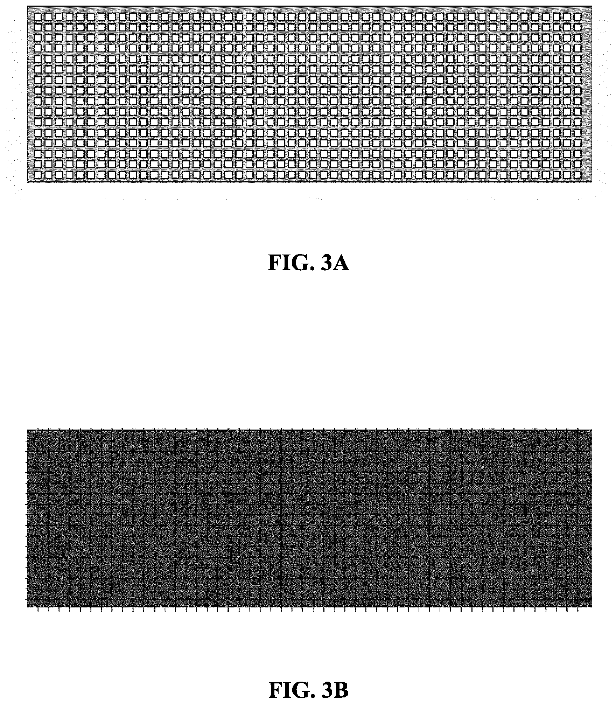

The diaphragm 19 is used to transfer and dissolve gas in a bubble-free manner into the mixture of animal cells and culture medium (or other liquids). The diaphragm 19 is arranged on the outer surface of the wall 12 and covers the ventilation slot 13. The diaphragm 19 may be fixed to the wall 12 by bonding, welding, or other means, such as the diaphragm 19 may be fixed to the radially outer surfaces of the top flange 14, the bottom flange 15, and/or of the protrusions 16 of the wall 12. The diaphragm 19 may be made of silica gel, polydimethylsiloxane (PDMS), polycarbonate track-etch (PCTE), polyethylene terephthalate (PETE), polytetrafluoroethylene (PTFE), polypropylene (PP), polycarbonate (PC), nylon, polyethersulfone (PES), sintered porous material, or the like. The diaphragm 19 may be processed with hydrophilic and positive charge or negative charge treatment so that it is not easy to be blocked due to being adsorbed by animal cells. As shown in FIG. 3A, the diaphragm 19 may be in a form of a microporous membrane. The microporous membranes may adopt a variety of pore sizes according to different biological processes (such as stem cells, tumor cells, CHO cells, or microcarrier processes) The pore sizes are less than 0.05 $\mu$m, for example, including but not limited to 0.01$\mu$m, 0.05um, 0.10$\mu$m, 0.20$\mu$m, 0.04$\mu$m, etc., and under the above-mentioned pore size conditions, the diaphragm 19 may not produce bubbles when ventilated under a certain pressure. In addition, as shown in FIG. 3B, the diaphragm 19 may also be in a form of a dense membrane. The dense membrane may be made of, for example, a silica gel material of PDMS. The dense membrane has no concept of pore sizes, but it is required to be as thin as possible in thickness, such as 50 $\mu$m to 500 $\mu$m.

Back to FIG. 1, the gas inlet unit 20 is connected to the gas inlet 17 through a gas inlet pipeline 21 and is arranged outside the tank 2. The gas inlet unit 20 may transmit the gas from the outside to the ventilation slot 13 in a controlled manner. The gas inlet unit 20 may include a gas flow component regulator 22 (such as a mass flowmeter or a gas-proportion regulating valve) arranged on the gas inlet pipeline 21. The gas flow component regulator 22 may regulate the flow and component ratio of inlet gas. In this embodiment, the inlet gas may be mainly composed of air, oxygen and carbon dioxide, or mainly composed of nitrogen, oxygen and carbon dioxide. A sum of the overall flow of the inlet gas is a fixed value, that is, a sum of the flow of air, oxygen, and carbon dioxide is a fixed value, or a sum of the flow of nitrogen, oxygen, and carbon dioxide is a fixed value. The gas flow component regulator 22 is controlled by a controller to maintain the total flow of inlet gas at the fixed value and adjust the proportion of oxygen in the total flow in real-time, so as to adjust the dissolved oxygen concentration in the mixture.

The gas outlet unit 30 is connected to the gas outlet 18 through a gas outlet pipeline 31 and is arranged outside the tank 2. The gas outlet unit 30 may discharge the undissolved gas in the ventilation slot 13 in a controlled manner to prevent the pressure increase in the gas distributor 10 from affecting the gas control of the gas inlet unit 20. The gas outlet unit 30 may include a dissolved oxygen electrode (not shown) provided in the gas outlet pipeline 31. The dissolved oxygen electrode may be used to detect a dissolved oxygen concentration value in the gas outlet pipeline 31 and feed it back to the controller. The gas outlet unit 30 may also include a pressure control valve and a sensor arranged in the gas outlet pipeline 31 to control the gas pressure (generally maintained between 0.01mpa-0.1mpa) in the ventilation slot 13 of the gas distributor 10, so as to maintain mass transfer efficiency of the diaphragm 19.

The solution monitoring unit 40 may include a dissolved oxygen electrode 41 and a pH electrode 42 electrically connected to the controller. The dissolved oxygen electrode 41 and the pH electrode 42 may be arranged in a mixture of cells and culture medium (or other liquids) of the tank 2. The dissolved oxygen electrode 41 is used to detect the dissolved oxygen concentration value of the mixture and feed it back to the controller. The pH electrode 42 is used to detect the pH value of the mixture and feed it back to the controller.

The controller may monitor the oxygen uptake rate (OUR) consumed by animal cells in real-time through the dissolved oxygen concentration value detected by the dissolved oxygen electrode of the gas outlet unit 30 and the dissolved oxygen concentration value detected by the dissolved oxygen electrode 41 of the solution monitoring unit 40, and adjusts the oxygen proportion in the inlet gas of the gas flow component regulator 22 of the gas inlet unit 20. For example, the dissolved oxygen of the mixture is set to no less than 40% during a culture process, with the increase of the number of animal cells in the mixture, the oxygen flow also needs to be increased, and the proportion in the total gas flow also increases in order to maintain the same dissolved oxygen. In addition, the controller may adjust the proportion of carbon dioxide in the inlet gas of the gas flow component regulator 22 of the gas inlet unit 20 through the pH value detected by the pH electrode 42 of the solution monitoring unit 40, so as to adjust the pH value of the mixture. For example, the pH value of the mixture is set to a fixed value during the culture process, with the continuous increase of pH value during the culture process, in order to maintain a constant pH value, the flow rate of carbon dioxide also needs to be increased, and the proportion in the total gas flow also increases.

According to the ventilation device 1 of the present disclosure, OUR can be detected more accurately. The ventilation device 1 adopts the design of the microporous membrane or the dense membrane, and the gas is discharged through the ventilation device 1, there is no tail gas buffer zone in the upper non-liquid area of the bioreactor. Therefore, the error in the part of OUR calculation may be reduced. The dissolved oxygen electrode of the gas outlet unit 30 may detect the dissolved oxygen concentration value Cout in the gas outlet pipeline 31 of the gas outlet unit 30, and the dissolved oxygen electrode 41 of the solution monitoring unit 40 may detect the dissolved oxygen concentration value Csensor of the mixture in the tank 2. Therefore, the oxygen uptake rate OUR in tank 2 of the bioreactor is obtained by the following formula:

$$OUR = (Cout - Csensor) * KLa,$$

where KLA is the mass transfer coefficient of the gas distributor 10.

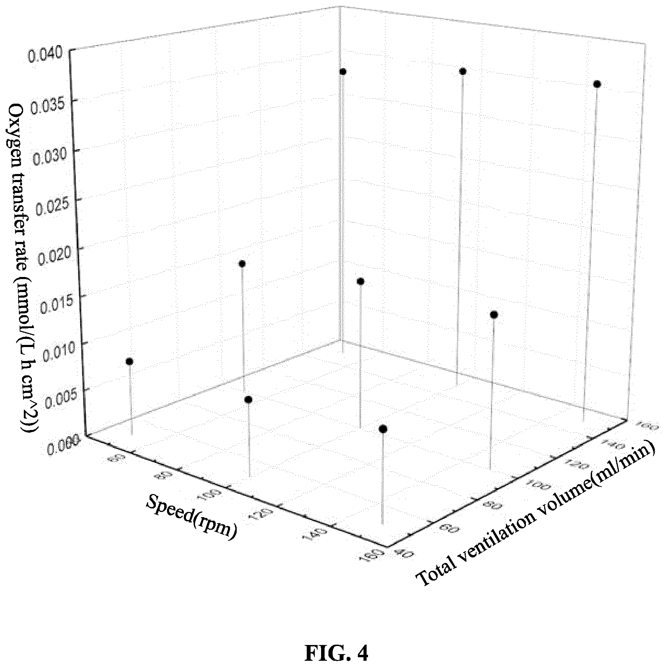

The mass transfer coefficient KLA is related to the motor speed driving the circulating flow of the mixture in tank 2, the total ventilation volume of the gas distributor 10, the bubble sizes in the tank 2, and other parameters. The ventilation device 1 according to the present disclosure adopts a bubble-free design, so no bubbles may be generated in the tank 2. FIG. 4 is a schematic diagram (taking 100um PDMS silica gel film under atmospheric pressure as an example) illustrating an exemplary relationship between the oxygen transfer rate (a parameter proportional to mass transfer coefficient KLA), motor speed, and total ventilation volume. Since the ventilation device 1 adopts a ventilation mode of equal ventilation volume, the total ventilation volume of the gas distributor 10 remains unchanged no matter how the process changes. Therefore, the mass transfer coefficient KLA of the gas distributor 10 is only related to the motor speed. The controller of the ventilation device 1 may build the relationship between the mass transfer coefficient KLA and the motor speed in a preset program, and the motor speed may not change significantly in a short time. Therefore, the ventilation device 1 of the present disclosure may measure the mass transfer coefficient KLA of the gas distributor 10 more accurately in advance.

Therefore, the controller may calculate the parameter OUR through the dissolved oxygen concentration values detected by the dissolved oxygen electrode of the gas outlet unit 30 and the dissolved oxygen electrode 41 of the solution monitoring unit 40, and display a calculation result to an operator in real-time. The parameter OUR is closely related to the growth state and biomass of animal cells. These parameters may be combined with the results of cell count or living cell electrode to form key parameters such as the oxygen consumption rate of a single animal cell, which is very important for bioreactors.

The mass transfer coefficient KLA of the ventilation device 1 according to the present disclosure is less affected by other factors, so it can be measured more accurately in advance. In addition, the total gas input of the ventilation device 1 according to the present disclosure remains basically unchanged. Therefore, OUR may be obtained directly through a change of oxygen intake, which greatly improves the calculation accuracy of OUR.

According to the ventilation device 1 of the present disclosure, the dissolved oxygen electrode may be used instead of an expensive tail gas mass spectrometer detector to calculate the oxygen consumption rate, which greatly improves the monitoring level of the bioreactor for the oxygen consumption rate in the biological process and reduces the procurement cost of the expensive mass spectrometer detector.

The ventilation device 1 according to the present disclosure uses a microporous membrane or a dense membrane to dissolve carbon dioxide, which is more conducive to the transmission effect of carbon dioxide. According to the ventilation device 1 of the present disclosure, there will be no phenomenon that in the bubble ventilation mode, the carbon dioxide cannot be discharged in an area with high viscosity, resulting in high local carbon dioxide concentration and carbon dioxide poisoning in animal cells.

The ventilation device 1 according to the present disclosure can be used in a disposable plastic bioreactor, which does not need damp-heat sterilization and only needs radiation sterilization. Therefore, the disposable plastic reactor can use a new microporous membrane material to improve the effect of oxygen transfer and achieve bubble-free mass transfer. The ventilation device 1 according to the present disclosure fills the gap of this design.

Although the exemplary embodiments of the present disclosure have been described, those skilled in the art should understand that various changes and amendments can be made to the exemplary embodiments of the present disclosure without departing from the spirit and scope of the present disclosure in essence. Therefore, all changes and amendments contained in the present disclosure are within the scope of protection. The present disclosure is defined by additional claims, and equivalents of these claims are also included.

**Claims**

1. A ventilation device for a bioreactor, wherein the bioreactor has a tank containing a mixture of animal cells and liquids, and the ventilation device comprises:

   a gas distributor, which is configured to be immersed in the mixture of the tank and includes a distributor body and a diaphragm supported by the distributor body, wherein the distributor body is provided with a ventilation slot, and the diaphragm is configured to dissolve a gas in the ventilation slot into the mixture without bubbles;

   a gas inlet unit, which is in fluid communication with the ventilation slot and transmits an inlet gas to the ventilation slot; and
   a gas outlet unit, which is in fluid communication with the ventilation slot and discharges an undissolved gas in the ventilation slot from the distributor body.

2. The ventilation device according to claim 1, wherein the ventilation slot is arranged on a wall of the distributor body and is configured to guide the inlet gas to flow on an entire outer surface of the wall.

3. The ventilation device according to claim 2, wherein the wall includes a top flange, a bottom flange, and one or more protrusions between the top flange and the bottom flange, and the top flange, the bottom flange, and the protrusions all protrude radially outward from an outer surface of the wall.

4. The ventilation device according to claim 3, wherein the top flange, the bottom flange, and/or the protrusions form a support frame supporting the diaphragm.

5. The ventilation device according to claim 3, wherein the ventilation slot is formed in a space between the outer surface of the wall and a side surface of adjacent protrusions, and/or in a space between the outer surface of the wall, a side surface of the protrusions and a side surface of the top flange, and/or in a space between the outer surface of the wall, the side surface of the protrusions and a side surface of the bottom flange.

6. The ventilation device according to claim 2, wherein the ventilation slot is in fluid communication with the gas inlet unit through a gas inlet of the distributor body and is in fluid communication with the gas outlet unit through a gas outlet of the distributor body.

7. The ventilation device according to claim 6, wherein the gas inlet and the gas outlet are tubular.

8. The ventilation device according to claim 2, wherein the ventilation slot presents a serpentine shape winding mainly along a horizontal direction on the entire outer surface of the wall.

9. The ventilation device according to claim 2, wherein the ventilation slot presents a serpentine shape winding mainly along a vertical direction on the entire outer surface of the wall.

10. The ventilation device according to claim 2, wherein the ventilation slot presents a spiral shape extending from bottom to top around the entire outer surface of the wall.

11. The ventilation device according to any one of claims 2-10, wherein the diaphragm is arranged on the outer surface of the wall and covers the ventilation slot.

12. The ventilation device according to any one of claims 3-5, wherein the diaphragm is fixed to radial outer surfaces of the top flange, the bottom flange, and/or the protrusions of the wall.

13. The ventilation device according to any one of claims 1-10, wherein the diaphragm is in a form of a dense membrane or a microporous membrane.

14. The ventilation device according to claim 13, wherein a pore size of the microporous membrane is configured to ventilate the diaphragm without bubbles under a certain pressure.

15. The ventilation device according to claim 14, wherein the pore size of the microporous membrane is less than 0.05 $\mu$m.

16. The ventilation device according to claim 14, wherein the pore size of the microporous membrane is one or more of 0.01 $\mu$m, 0.05 um, 0.10 $\mu$m, 0.20 $\mu$m, and 0.04 $\mu$m.

17. The ventilation device according to claim 13, wherein a thickness of the dense membrane is between 50 $\mu$m to 500 $\mu$m.

18. The ventilation device according to any one of claims 1-10, wherein the diaphragm is made of silica gel, polydimethylsiloxane (PDMS), polycarbonate track-etch (PCTE), polyethylene terephthalate (PETE), polytetrafluoroethylene (PTFE), polypropylene (PP), polycarbonate (PC), nylon, polyethersulfone (PES), or sintered porous material.

19. The ventilation device according to any one of claims 1-10, wherein the diaphragm is processed with hydrophilic and positive charge or negative charge treatment so that it is not easy to be blocked due to being adsorbed by animal cells.

20. The ventilation device according to claim 1, wherein the gas inlet unit is in fluid communication with the ventilation slot through a gas inlet pipeline, the gas inlet unit includes a gas flow component regulator arranged on the gas inlet pipeline and electrically connected to a controller, the gas flow component regulator is configured to maintain a total flow of the inlet gas at a constant value and adjust a component proportion of the inlet gas according to a command of the controller.

21. The ventilation device according to claim 20, wherein the gas flow component regulator is any one of a mass flowmeter or a gas-proportion regulating valve.

22. The ventilation device according to claim 20, wherein the inlet gas includes air, oxygen, and carbon dioxide, or includes nitrogen, oxygen, and carbon dioxide.

23. The ventilation device according to claim 20, wherein the gas outlet unit is in fluid communication with the ventilation slot through a gas outlet pipeline, the gas outlet unit includes a first dissolved oxygen electrode arranged in the gas outlet pipeline and electrically connected to the controller, and the first dissolved oxygen electrode is configured to detect a dissolved oxygen concentration value in the gas outlet pipeline.

24. The ventilation device according to claim 20, wherein the gas outlet unit further includes a pressure control valve and a sensor arranged in the gas outlet pipeline and electrically connected to the controller to control gas pressure in the ventilation slot.

25. The ventilation device according to claim 24, wherein the gas pressure in the ventilation slot is maintained between 0.01Mpa-0.1Mpa.

26. The ventilation device according to claim 23, wherein the ventilation device further includes a solution monitoring unit, which includes a second dissolved oxygen electrode arranged in the mixture and electrically connected to the controller, the second dissolved oxygen electrode being configured to detect the dissolved oxygen concentration value in the mixture.

27. The ventilation device according to claim 26, wherein the solution monitoring unit includes a pH electrode arranged in the mixture and electrically connected to the controller, and the pH electrode is configured to detect a pH value in the mixture.

28. The ventilation device according to claim 26, wherein the controller is configured to monitor and adjust an oxygen uptake rate OUR consumed by the animal cells in the tank in real-time through the dissolved oxygen concentration value Cout detected by the first dissolved oxygen electrode and the dissolved oxygen concentration value Csensor detected by the second dissolved oxygen electrode.

29. The ventilation device according to claim 28, wherein the oxygen uptake rate OUR consumed by the animal cells in the tank is determined by a calculation formula of

$$OUR = (Cout-Csensor) \cdot KLa,$$

where KLA is a mass transfer coefficient of a gas distributor.

30. The ventilation device according to claim 29, wherein the mass transfer coefficient of the gas distributor is related to a rotational speed of a motor in the tank, the motor driving the circulating flow of the mixture.

31. The ventilation device according to claim 27, wherein the controller is configured to adjust a proportion of carbon dioxide in the inlet gas of the gas flow component regulator through a pH value detected by a pH electrode, so as to adjust a pH value of the mixture.

32. A ventilation device for a bioreactor, wherein the bioreactor has a tank containing a mixture of animal cells and liquid, and the ventilation device comprises:

a gas distributor, which is configured to be immersed in the mixture of the tank and is configured to dissolve an inlet gas into the mixture without bubbles;

a gas inlet unit, which is in fluid communication with the gas distributor and transmits an inlet gas to the gas distributor, the gas inlet unit including a gas flow component regulator, wherein the gas flow component regulator is configured to maintain a total flow of the inlet gas at a constant value and adjust the component proportion of the inlet gas;

a gas outlet unit, which is in fluid communication with the gas distributor and discharges an undissolved gas in the gas distributor from the distributor body, wherein the gas outlet unit includes a first dissolved oxygen electrode configured to detect a dissolved oxygen concentration value in the undissolved gas;

a solution monitoring unit, which includes a second dissolved oxygen electrode configured to detect the dissolved oxygen concentration value in the mixture; and

a controller, which is configured to monitor and adjust an oxygen uptake rate OUR consumed by animal cells in the tank in real-time through the dissolved oxygen concentration value Cout detected by the first dissolved oxygen electrode and the dissolved oxygen concentration value Csensor detected by the second dissolved oxygen electrode.

33. The ventilation device according to claim 32, wherein the gas flow component regulator is any one of a mass flowmeter or a gas-proportion regulating valve.

34. The ventilation device according to claim 32, wherein the inlet gas includes air, oxygen, and carbon dioxide, or includes nitrogen, oxygen, and carbon dioxide.

ide.

35. The ventilation device according to claim 32, wherein the gas outlet unit further comprises a pressure control valve configured to control gas pressure in the gas distributor and a sensor.

36. The ventilation device according to claim 35, wherein the gas pressure in the gas distributor is maintained between 0.01Mpa-0.1Mpa.

37. The ventilation device according to claim 32, wherein the solution monitoring unit comprises a pH electrode arranged in the mixture and electrically connected to the controller, and the pH electrode is configured to detect the pH value in the mixture.

38. The ventilation device according to claim 32, wherein the oxygen uptake rate OUR consumed by the animal cells in the tank is determined by a calculation formula of

$$OUR = (Cout - Csensor) * KLa,$$

where KLA is a mass transfer coefficient of the gas distributor.

39. The ventilation device according to claim 38, wherein the mass transfer coefficient of the gas distributor is related to a rotational speed of a motor in the tank which drives the circulating flow of the mixture.

40. The ventilation device according to claim 37, wherein the gas controller is configured to adjust a proportion of carbon dioxide in the inlet gas of the gas flow component regulator through a pH value detected by a pH electrode, so as to adjust a pH value of the mixture.

41. The ventilation device described in any of claims 32-40 wherein the gas distributor includes a distributor body and a diaphragm supported by the distributor body, the distributor body being provided with a ventilation slot, and the diaphragm being configured to dissolve a gas in the ventilation slot into the mixture without bubbles;

42. The ventilation device according to claim 41, wherein the diaphragm is in a form of a dense membrane or a microporous membrane.

43. The ventilation device according to claim 42, wherein a pore size of the microporous membrane is configured to ventilate the diaphragm without bubbles under a certain pressure.

44. The ventilation device according to claim 43, wherein the pore size of the microporous membrane is less than 0.05 µm.

45. The ventilation device according to claim 43, wherein the pore size of the microporous membrane is one or more of 0.01 µm, 0.05 um, 0.10 µm, 0.20 µm, and 0.04 µm.

46. The ventilation device according to claim 42, wherein a thickness of the dense membrane is between 50 µm to 500 µm.

47. The ventilation device according to any one of claims 32-40, wherein the diaphragm is made of silica gel, polydimethylsiloxane (PDMS), polycarbonate track-etch (PCTE), polyethylene terephthalate (PETE), polytetrafluoroethylene (PTFE), polypropylene (PP), polycarbonate (PC), nylon, polyethersulfone (PES), sintered porous material, or the like.

48. The ventilation device according to any one of claims 32-40, wherein the diaphragm is processed with hydrophilic and positive charge or negative charge treatment so that it is not easy to be blocked due to being adsorbed by animal cells.

**FIG. 1**

**FIG. 2**

**FIG. 3A**

**FIG. 3B**

**FIG. 4**

## INTERNATIONAL SEARCH REPORT

| International application No. |
| --- |
| **PCT/CN2021/073796** |

### A.　CLASSIFICATION OF SUBJECT MATTER

C12M 3/00(2006.01)i;　C12M 1/34(2006.01)i;　C12M 1/12(2006.01)i;　C12M 1/04(2006.01)i

According to International Patent Classification (IPC) or to both national classification and IPC

### B.　FIELDS SEARCHED

Minimum documentation searched (classification system followed by classification symbols)

　　C12M

Documentation searched other than minimum documentation to the extent that such documents are included in the fields searched

Electronic data base consulted during the international search (name of data base and, where practicable, search terms used)

　　CNABS; CNMED; CNKI; CNTXT; 百度学术: 生物反应器, 罐, 分布器, 膜, 气泡 et al.; DWPI; SIPOABS; EPTXT; USTXT; WOTXT; JPTXT; PUBMED; ISI WEB OF SCIENCE: bioreactor?, tank?, distributer?, distributor?, sparger?, diaphragm?, film? membrane?, tunic?, velum?, blister?, bubble? et al.

### C.　DOCUMENTS CONSIDERED TO BE RELEVANT

| Category* | Citation of document, with indication, where appropriate, of the relevant passages | Relevant to claim No. |
| --- | --- | --- |
| X | CN 106754259 A (INSTITUTE OF PROCESS ENGINEERING, CHINESE ACADEMY OF SCIENCES) 31 May 2017 (2017-05-31)<br>　　see description, paragraphs [0013]-[0140], figures 1-2 | 1-25 |
| Y | CN 106754259 A (INSTITUTE OF PROCESS ENGINEERING, CHINESE ACADEMY OF SCIENCES) 31 May 2017 (2017-05-31)<br>　　see description, paragraphs [0013]-[0140], figures 1-2 | 26-48 |
| Y | CN 109620597 A (JINAN KUNZHONG INFORMATION TECHNOLOGY CO., LTD. et al.) 16 April 2019 (2019-04-16)<br>　　see description, paragraphs [0003]-[0096], figures 1-4 | 26-48 |
| PX | CN 111454841 A (SHANGHAI AIZHONG BIO-TECH CO., LTD.) 28 July 2020 (2020-07-28)<br>　　see description, paragraphs [0006] to [0098] | 1-48 |
| A | CN 111117862 A (SHANGHAI AIZHONG BIO-TECH CO., LTD.) 08 May 2020 (2020-05-08)<br>　　see description, specific embodiments | 1-48 |

☑ Further documents are listed in the continuation of Box C.　　☑ See patent family annex.

| | |
| --- | --- |
| *　Special categories of cited documents:<br>"A"　document defining the general state of the art which is not considered to be of particular relevance<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| Date of the actual completion of the international search | Date of mailing of the international search report |
| --- | --- |
| **30 March 2021** | **26 April 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/073796** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 208898908 U (SUZHOU DIOUYI BIOLOGICAL TECHNOLOGY CO., LTD.) 24 May 2019 (2019-05-24)<br>see description, specific embodiments | 1-48 |

Form PCT/ISA/210 (second sheet) (January 2015)

**INTERNATIONAL SEARCH REPORT**
**Information on patent family members**

International application No.

**PCT/CN2021/073796**

| Patent document cited in search report | | | Publication date (day/month/year) | Patent family member(s) | Publication date (day/month/year) |
|---|---|---|---|---|---|
| CN | 106754259 | A | 31 May 2017 | None | |
| CN | 109620597 | A | 16 April 2019 | None | |
| CN | 111454841 | A | 28 July 2020 | None | |
| CN | 111117862 | A | 08 May 2020 | None | |
| CN | 208898908 | U | 24 May 2019 | None | |

Form PCT/ISA/210 (patent family annex) (January 2015)